# EUROPEAN PATENT SPECIFICATION

(11) **EP 2 225 035 B1**
(45) Date of publication and mention of the grant of the patent: **25.04.2018**
(21) Application number: 08859059.1
(22) Date of filing: 03.12.2008
(51) Int. Cl.: B01J 38/74, C07C 1/24, B01J 23/30, B01J 38/68

(54) **CATALYST RECOVERY PROCESS**
VERFAHREN ZUR KATALYSATORRÜCKGEWINNUNG
PROCEDE DE RECUPERATION DE CATALYSEUR

(30) Priority: 13.12.2007 EP 07254848
(43) Date of publication of application: 08.09.2010
(73) Proprietor: Technip E&C Limited, Milton Keynes, Buckinghamshire MK9 1BA (GB)
(72) Inventor: CHIRON, Francois-Xavier, Saint-Laurent Quebec H4L 2E1 (CA); FULLERTON, William, Keyingham East Riding of Yorkshire HU12 9SL (GB); KEY, Leslie, Ann, Hull East Riding of Yorkshire HU12 8QH (GB); O'NEILL, Liam, West Yorkshire HD8 8XU (GB); SMITH, Stephen, James, Cottingham East Yorkshire HU16 5LL (GB)
(74) Representative: Stott, Michael John
(86) International application number: PCT/GB2008/004014
(87) International publication number: WO 2009/074774

(56) References cited:
- WO-A-2005/107945
- WO-A-2007/003899
- JP-A- 56 163 755
- US-A- 2 968 527
- US-A- 5 716 895

## Description

The present invention relates to a process for the recovery of heteropolyacids from catalysts used for a variety industrial scale uses. In particular the invention relates to the recovery of heteropolyacids containing tungsten from spent industrial catalysts in which the heteropolyacid is supported upon a carrier or support.

Heteropolyacids are widely used in the chemical industry to catalyse a range of industrial processes which require the presence of a strong acid. Examples include the isomerisation of paraffins (US provisional patent application 2002/0023859), the preparation of N-acetyl aminophenols (US Patent 5387702), the preparation of bis-phenols, the production of ethyl benzene or cumene, and the dehydration of alcohols and alkoyalkanes to produce alkenes.

In processes such as these the catalyst inevitably has a finite lifetime after which they become spent. In other words it is no longer either technically or economically viable to use them. One common reason why such catalysts become spent over time is the lay-down of organic degradation products (usually referred to as 'coke') on the surface of the catalyst or within its pores if the support itself is porous. The problems of said coking can manifests themselves in a variety of undesirable ways including reduced activity (i.e. feedstock conversion at a given temperature) and reduced product selectivity (i.e. an increased production of undesirable by-products). For example, when silica supported silicotungstic acid catalysts are use to effect the dehydration of ethanol or ethoxyethane progressive coking overtime leads to an increase in by-product ethane make at the expense of the desired ethene.

A second reason why supported heteropolyacids lose their effectiveness over time is the build up of metals on their surface and in their pores. These metals which arise from impurities present in the feedstock and slow corrosion of the equipment in which the process is carried out are generally present in small but nonetheless significant amounts. For example in the production of ethene by the process referred to above build up of cobalt, chromium, nickel; and iron can eventually lead to a significant make of by-product ethane.

Once a supported heteropolyacid catalyst is spent it is desirable for economic reasons to recover the heteropolyacid from the support. This is especially the case for catalysts involving heteropolytungstic acids on account of the high price of tungsten and tungsten compounds. The process disclosed in the present application therefore allows the efficient recovery of heteropolytungstic acids from spent catalysts thereby avoiding the time and cost associated with generating fresh catalysts from new sources of tungsten.

US Patent 5716895 discloses a process for regenerating heteropolymolybdic catalysts by dissolving them in an aqueous medium, oxidising the solution with hydrogen peroxide and treating the product with an inorganic ion-exchange material e.g. crystalline antimonic acid. The catalyst disclosed in this reference is however used for a completely different purpose, reactions of methacrolein and contain no tungsten. In addition use of the antimonic acid causes undesirable environmental issues as well as giving rise to the potential contamination of the final catalyst. WO 2007/003899 discloses the process for which our catalysts are used. WO 2005/107945 discloses a process for recovering corrosion metals from precious metal containing solutions using a cation-exchange resin. US 2968527 discloses recovering tungstic acid from aqueous media using an anion-exchange resin followed by elution with chloride ion. JP 56163755 discloses a process for recovering molybdophosphoric acids from spent catalyst by aqueous extraction and heat treatment in an oxygen containing atmosphere.

According to the present invention there is provided a process for recovering heteropolytungstic acid from a spent catalyst comprising a supported heteropolytungstic acid characterised in that the process comprises:
(a) contacting the spent catalyst with an extractant selected from water, methanol, ethanol or a mixture of any two or more thereof for sufficient time to extract at least part of the heteropolytungstic acid therefrom;
(b) separating the extractant containing heteropolytungstic acid from the treated spent catalyst;
(c) contacting the extractant containing heteropolytungstic acid with a strong acid ion exchange resin to remove nickel, cobalt, chromium and iron which have been leached off the spent catalyst in step (a) contained therein and
(d) recovering the treated extractant containing heteropolytungstic acid for subsequent use.

It has been found that fresh catalysts produced from heteropolytungstic acids recovered by the above-mentioned process have the same performance characteristics as equivalent catalysts produced from completely new and independent sources of the heteropolytungstic acid. The process of the present invention therefore minimises the need to purchase and use anything more than a minimum amount of extra tungsten or tungsten compounds. Furthermore by essentially recycling previously used tungsten the process leads to important environmental benefits.

In a preferred embodiment of the present invention the spent catalyst is washed with an organic liquid prior to step (a) in order to remove any 'soft' coke (readily soluble organic material) present thereon. By doing so removal of the heteropolytungstic acid in step (a) is facilitated. Although the organic liquid can be any organic compound in principle it is preferable to use commonly available solvents such as a C₄ to C₁₂ alkane (preferably a C₆ to C₁₀ alkane) or a mixture thereof or a liquid aromatic compound such as toluene or one or more of the xylenes. Alternatively some of the more volatile organics present in the coke may be purged from the spent catalyst by treating it in situ in the reactor before removal with hot nitrogen gas, steam or a mixture thereof before extraction.

In step (a) of the process of the present invention the spent catalyst is treated with an extractant selected from water, methanol, ethanol or a mixture of any two or more thereof. Treatment can be effected by contacting the spent catalyst with a continuous stream of extractant in a packed column. Alternatively the extractant and spent catalyst can be intimately mixed together with agitation to generate a slurry in which extraction occurs. Step (a) may be carried out at room temperature but it is preferable to accelerate the process by carrying it out at an elevated temperature suitably in the range from 30° to 100°C preferably from 40° to 85°C.

The extractant and spent catalyst are contacted together until it is found that no further heteropolytungstic acid can be removed under the conditions of contacting. This can easily be established for a given temperature by periodically withdrawing a sample of the mixture, separating the extractant and measuring the concentration of tungsten therein using conventional quantitative analytical techniques.

Of the extractants disclosed water is generally preferred on account of its ability to remove more heteropolytungstic acid from the spent catalyst and its non-flammability. Compared to ethanol the use of water is advantageous as it does not dissolve the coke. However the use of ethanol allows the removal of pore blockage coke and can consequently improve the heteropolytungstic acid recovery. For these reasons it can be advantageous to use a mixture of ethanol and water when treating large volumes of catalyst.

In step (b) of the process of the present invention the extractant containing the heteropolytungstic acid is separated from the treated spent catalyst using any methods practiced by those skilled in the art for separation of a liquid from a solid on a large scale. This can typically be by filtration (e.g. vacuum filtration, centrifugal filtration) or in the case of a slurry by decantation. In the event that the extractant after separation contains significant amount of organic material it may be necessary to remove this by liquid-liquid extraction using an alkane of the type referred to above which is immiscible with the extractant. Addition of water can be used to promote the separation.

The extractant containing the heteropolytungstic acid produced in step (b) will generally contain significant amounts of corrosion metals which have been leached off the spent catalyst in step (a) above. These corrosion metals are nickel, cobalt, chromium and iron i.e. the typical constituents of steel. It is important that the levels of these metals are reduced significantly before the heteropolytungstic acid is reused if the catalyst prepared with these materials from the process described herein is to show optimum performance characteristics.

If the heteropolytungstic acid is to be used to manufacture catalyst for the dehydration of ethanol or ethoxyethane to ethene then it is important that the molar ratio of total corrosion metal to heteropolytungstic acid is less than 0.25 to 1. To avoid confusion the heteropolytungstic acid is assumed to be fully hydrated when calculating the molecular weight. For the various corrosion metals it is also preferred that the individual molar ratios are as follows: chromium less than 0.22 to1; iron, less than 0.15 to 1; nickel less than 0.1 to 1 and cobalt less than 0.8 to 1. Preferably the molar ratio of total corrosion metals to heteropolytungstic acid is less than 0.15 to 1 most preferably less than 0.1 to 1. The corrosion metals are removed in step (c) of the process which comprises contacting the extractant containing heteropolytungstic acid with a strong acid ion exchange resin. This can take place by contacting the extractant containing the heteropolytungstic acid with a strong acid cation-exchange resin. A typical example of such a resin is the Amberlyst© family of resins manufactured by Rohm and Haas e.g. Amberlyst 15H or Amberlyst 35H resin. Resins similar in specification to these resins and manufactured by others e.g. Purolite© C145H can also be used. In a like manner to step (a), step (c) can be carried out in a fixed bed or in a slurry at a temperature in the range room temperature to 100°C. For water extractant the preferred temperature range is more than 60°C and less than 90°C at atmospheric pressure. For ethanol extractant the preferred temperature range is less than 60°C at atmospheric pressure. It may be advantageous to conduct this under pressure particularly when the tungsten from a fixed bed is to be recovered or if a volatile solvent is to be used. Progress of the removal of the corrosion metals over time at a given temperature and conditions can be followed by sampling and quantitative analysis to determine the optimum contact time for the ion-exchange such that the low residual levels referred to above are met.

The extractant containing heteropolytungstic acid from step (c) can in step (d) be used directly for manufacturing fresh catalyst (e.g. by contacting the solution with fresh support). Alternatively if this product is too dilute it can first be concentrated by partial removal of the extractant and if desired all the extractant can be removed to produce solid heteropolytungstic acid.

The term heteropolytungstic acid as used herein includes both the free acids themselves and soluble salts thereof. Said salts include inter alia; alkali metals, alkali earth metals, ammonium, as counter ions and/or transition metal salts (where the salts may be either full or partial salts), of heteropolytungstic acids. The heteropolytungstic acids referred to in the present invention are complex, high molecular weight anions comprising oxygen-linked metal atoms.

Typically, each anion comprises 12-18, oxygen-linked tungsten atoms. These atoms surround one or more of central atoms in a symmetrical manner. The central atoms are preferably silicon or phosphorus, but may alternatively comprise any one of a large variety of atoms from Groups I-VIII in the Periodic Table of elements. These include copper, beryllium, zinc, cobalt, nickel, boron, aluminium, gallium, iron, cerium, arsenic, antimony, bismuth, chromium, rhodium, silicon, germanium, tin, titanium, zirconium, vanadium, sulphur, tellurium, manganese nickel, platinum, thorium, hafnium, cerium, arsenic, vanadium, antimony ions, tellurium and iodine. Suitable heteropolytungstic acids include Keggin, Wells-Dawson and Anderson-Evans-Perloff heteropolytungstic acids. Specific examples of suitable heteropolytungstic acids are as follows:

| | |
|---|---|
| 18-tungstophosphoric acid | - H6[P2WO62].xH2O |
| 12-tungstophosphoric acid | - H3[PW12O40].xH2O |
| 12-tungstosilicic acid | - H4[SiW12O40].xH2O |
| Lithium hydrogen tungstosilicate | - Li3H[SiW12O40].xH2O |

and the free acid or partial salts of the following heteropolytungstic acids:

| | |
|---|---|
| Monopotassium tungstophosphate | - KH5[P2W18O62].xH2O |
| Monosodium 12-tungstosilicic acid | - NaK3[SiW12O40].xH2O |
| Potassium tungstophosphate | - K6[P2W18O62].xH2O |

In addition mixtures of different heteropolytungstic acids and salts can be present in the spent catalyst. The preferred ones in this respect are described by the present invention are any those based on the Keggin or Wells-Dawson structures; more preferably the chosen heteropolytungstic acid for use in the process described by the present invention is either: tungstosilicic acid, or tungstophosphoric acid. Most preferably the heteropolytungstic acid will be 12-tungstosilicic acid (H₄[SiW₁₂O₄₀].xH₂O).

Preferably, the heteropolytungstic acids employed will have molecular weights of more than 700 and less than 8500, preferably more than 2800 and less than 6000. Such heteropolytungstic acids also include dimeric complexes.

Suitable catalyst supports may be but are not limited to montmorillonite, clays, bentonite, diatomous earth, titania, activated carbon, alumina, silica-alumina, silica-titania cogels, silica-zirconia cogels, carbon coated alumina, zeolites (e.g. mordenite), zinc oxide, flame pyrolysed oxides. Supports can be mixed oxides, neutral or weakly basic oxides. Silica supports are preferred, such as silica gel supports and supports produced by the flame hydrolysis of SiCl₄. Preferred supports are substantially free of extraneous metals or elements which might adversely affect the catalytic activity of the system. Thus, suitable silica supports are at least 99% w/w pure. Impurities amount to less than 1% w/w, preferably less than 0.60% w/w and most preferably less than 0.30% w/w. The pore volume of the support is preferably more than 0.50ml/g and preferably more than 0.8 ml/g.

Suitable silica supports include, but are not limited to any of the following: Grace Davison Davicat® Grade 57, Grace Davison Davicat® 1252, Grace Davison Davicat® SI 1254, Fuji Silysia CariAct® Q15, Fuji Silysia CariAct® Q10, Degussa Aerolyst® 3045 and Degussa Aerolyst® 3043. The average diameter of the support particles is 2 to 10 mm, preferably 3 to 6 mm. However, these particles may be smaller, e.g. 0.5-2 mm, in some cases.

The average pore radius (prior to impregnation with the heteropolytungstic acid) of the support will generally be 10 to 500Å, preferably 30 to 175Å, more preferably 50 to 150 Å and most preferably 60 to 120Å. The BET surface area will generally be between 50 and 600 m2/g and most preferably between 150 and 400 m2/g. The support will generally have an average single particle crush strength of at least 1 kg force, suitably at least 2 kg force, preferably at least 6 kg force and more preferably at least 7 kg force. The bulk density of the support will generally be at least 380 g/l, preferably at least 395 g/l.

The single particle crush strength will be that determined by using a Mecmesin force gauge which measures the minimum force necessary to crush a particle between parallel plates. The crush strength is based on the average of that determined for a set of at least 25 catalyst particles.

The BET surface area, pore volume, pore size distribution and average pore radius will that be determined from the nitrogen adsorption isotherm determined at 77K using a Micromeritics TRISTAR 3000 static volumetric adsorption analyser. The procedure used will be an application of British Standard methods BS4359:Part 1:1984 'Recommendations for gas adsorption (BET) methods' and BS7591 :Part 2:1992, 'Porosity and pore size distribution of materials' - Method of evaluation by gas adsorption. The resulting data should be reduced using the BET method (over the pressure range 0.05-0.20 P/Po) and the Barrett, Joyner & Halenda (BJH) method (for pore diameters of 20-1000 Å) to yield the surface area and pore size distribution respectively.

Suitable references for the above data reduction methods are Brunauer, S, Emmett, P H, & Teller, E, J. Amer. Chem. Soc. 60, 309, (1938) and Barrett, E P, Joyner, LG & Halenda P P, J. Am Chem. Soc. , 1951 73 373-380.

A preferred heteropolytungstic acid supported catalyst which is suitable for treatment by the process of the present invention is one having the following characteristic:
PV > 0.6 - 0.3 x [HPA loading/Surface Area of Catalyst]
wherein PV is the pore volume of the dried supported heteropolytungstic acid catalyst (measured in ml/g catalyst); HPA loading is the amount of heteropolyacid present in the dried supported heteropolyacid catalyst (measured in micro moles per gram of catalyst) and Surface Area of Catalyst is the surface area of the dried supported heteropolytungstic acid catalyst (measured in m² per gram of catalyst).

The amount of heteropolytungstic acid impregnated onto the support will suitably be in the range of 10 wt % to 80 wt % and preferably in between 20 wt % to 50 wt %, based on the total weight of the heteropolytungstic acid and of the support.

The weight of the catalyst on drying and the weight of the support used, may be used to obtain the weight of the acid on the support by deducting the latter from the former, giving the catalyst loading as a 'g heteropolytungstic acid/kg catalyst' term. The catalyst loading in 'grams of heteropolytungstic acid /litre support' can also be calculated by using the known or measured bulk density, of the support. The preferred catalytic loading of heteropolytungstic acid will be 150 to 600g heteropolytungstic acid /kg catalyst and the average heteropolytungstic acid loading per surface area of the dried supported heteropolytungstic acid catalyst will be more than 0.1 micro moles/m². The amount of chloride present in/on the said heteropolytungstic acid supported catalyst will be less than 40 ppm, preferably less than 25 ppm and most preferably less than 20 ppm.

The process of the present invention although applicable on a commercial scale to a wide range of spent heteropolytungstic acid catalysts and is especially suitable for treating spent catalysts used in the conversion of alcohols and alkoyalkanes to alkenes to alkenes especially the conversion of ethanol, ethoxyethane and mixtures thereof containing water to ethene.

The present invention is now illustrated with reference to the following examples.

### Example 1

45g (73ml) of a spent silica supported silicotungstic acid catalyst (<4mm diameter) is placed in a fixed bed reactor .A continuous flow distilled water at 80°C is then passed though the fixed bed at an LHSV of 0.5hr⁻¹ for a period of six hours and the essentially colourless eluent collected. Analysis of this effluent shows that approximately 70% of the silicotungstic acid is recovered. The eluent is then passed though a fixed bed of Amberlyst 15H ion-exchange resin at the same temperature to remove any corrosion metals and the effluent is again collected. Solid silicotungstic acid is recovered essentially pure from this eluent using a rotary evaporator (at 70°C and less than 0.1MPa pressure) followed by subsequent drying at 100°C in an oven.

Typically the eluent from the first stage washing referred to above contains 11ppm iron, 2ppm chromium, 4ppm nickel and <2ppm each of molybdenum, manganese and copper. After treatment with the ion exchange resin the corrosion metals content of the eluent is 3ppm iron, and <2ppm chromium, nickel, molybdenum, manganese and copper.

### Example 2 and 3

Equivalent experiments to Example 1 in which the catalyst washing is carried out at 20°C (Example 2) and 50°C (Example 3) leads to the recovery of approximately 60 and 65% respectively of the silicotungstic acid.

### Example 4

Example 1 is repeated except that 45g (73ml) of spent silica supported silicotungstic acid (<4mm diameter) is placed in a fixed bed reactor and a continuous flow of absolute ethanol at an LHSV of 0.5hr⁻¹ for a period of six hours at 20°C is passed though the bed and the eluent collected. Analysis of the eluent shows that approximately 60% of the silicotungstic acid is recovered. The eluent is dark green/brown indicating the presence of dissolved organic matter. This organic matter is derived from the coke on the spent catalyst.

### Examples 5 to 21

Further experiments were carried out as detailed in the table below.

| Example | Solvent | Spent Catalyst Particle Size (mm) | Catalyst Weight (g) | Catalyst Volume (mls) | Temperature (°C) | LHSV (ml Solvent / ml catalyst / h-1) | Total Extraction time (hr) | Tungsten Recovered (%) |
|---|---|---|---|---|---|---|---|---|
| Example 1 | Water | <4 | 45 | 73 | 80 | 0.5 | 6 | 73 |
| Example 2 | Water | <4 | 45 | 73 | 20 | 0.5 | 6 | 61 |
| Example 3 | Water | <4 | 45 | 73 | 50 | 0.5 | 6 | 65 |
| Example 4 | Ethanol | <4 | 45 | 73 | 20 | 0.5 | 6 | 58 |
| Example 5 | Ethanol | <4 | 20 | 38 | 20 | 2 | 6 | 59 |
| Example 6 | Ethanol | <4 | 20 | 38 | 20 | 5 | 6 | 50 |
| Example 7 | Ethanol | <4 | 20 | 38 | 20 | 8 | 6 | 44 |
| Example 8 | Ethanol | <4 | 20 | 38 | 20 | 10 | 6 | 35 |
| Example 9 | Ethanol | <4 | 20 | 38 | 40 | 2 | 6 | 34 |
| Example 10 | Ethanol | <4 | 20 | 38 | 40 | 5 | 6 | 41 |
| Example 11 | Ethanol | <4 | 20 | 38 | 40 | 8 | 6 | 32 |
| Example 12 | Ethanol | <4 | 20 | 38 | 40 | 10 | 6 | 32 |
| Example 13 | Ethanol | <4 | 20 | 38 | 60 | 2 | 6 | 25 |
| Example 14 | Ethanol | <4 | 20 | 38 | 60 | 5 | 6 | 19 |
| Example 15 | Ethanol | <4 | 20 | 38 | 60 | 8 | 6 | 13 |
| Example 16 | Ethanol | <4 | 20 | 38 | 60 | 10 | 6 | 4 |
| Example 17 | Ethanol | <4 | 20 | 38 | 20 | 0.5 | 6 | 62 |
| Example 18 | Ethanol | <4 | 20 | 38 | 20 | 0.5 | 6 | 59 |
| Example 19 | Ethanol | <4 | 20 | 38 | 20 | 0.2 | 6 | 66 |
| Example 20 | Ethanol | <1 | 20 | 38 | 20 | 0.2 | 6 | 71 |
| Example 21 | Water | <1 | 45 | 73 | 80 | 0.5 | 6 | 80 |

## Claims

1. A process for recovering heteropolytungstic acid from a spent catalyst comprising a supported heteropolytungstic acid **characterised in that** the process comprises:
(a) contacting the spent catalyst with an extractant selected from water, methanol, ethanol or a mixture of any two or more thereof for sufficient time to extract at least part of the heteropolytungstic acid therefrom;
(b) separating the extractant containing heteropolytungstic acid from the treated spent catalyst;
(c) contacting the extractant containing heteropolytungstic acid with a strong acid ion exchange resin to remove nickel, cobalt, chromium and iron which have been leached off the spent catalyst in step (a) contained therein and
(d) recovering the treated extractant containing heteropolytungstic acid for subsequent use.

2. A process according to claim 1, wherein the heteropolyacid is tungstosilicic acid or tungstophosphoric acid.

3. A process as claimed in claim 1 or claim 2, **characterised in that** the spent catalyst is washed before step (a) with an organic liquid to remove soft coke.

4. A process as claimed in claim 1 **characterised in that** step (a) is carried out at a temperature in the range from 40° to 85°C.

5. A process as claimed in claim 1 **characterised in that** the extractant is a mixture of ethanol and water.

6. A process as claimed in claim 1 **characterised in that** the extractant is water

7. A process as claimed in claim 1 **characterised in that** the extractant is ethanol, and step (c) is preceded by a step in which soft coke dissolved in the extractant is removed using liquid-liquid extraction.

8. A process as claimed in claim 1 **characterised in that** the spent catalyst has been used to effect the dehydration of ethoxyethane, ethanol or mixtures thereof to form ethene.

9. A process as claimed in claim 1 **characterised in that** recovered heteropolytungstic acid is reutilised to make fresh supported heteropolytungstic acid.

10. A process as claimed in claim 3 characterised that the organic liquid is a C₆ to C₁₀ alkane or a mixture thereof.

## Patentansprüche

1. Verfahren zum Rückgewinnen von Heteropolywolframsäure aus einem erschöpften Katalysator, umfassend eine geträgerte Heteropolywolframsäure, **dadurch gekennzeichnet, dass** das Verfahren Folgendes umfasst:
(a) Inkontaktbringen des erschöpften Katalysators mit einem Extraktionsmittel, ausgewählt aus Wasser, Methanol, Ethanol oder einem Gemisch aus jedweden zwei oder mehr davon, für eine ausreichende Zeit, um mindestens einen Teil der Heteropolywolframsäure daraus zu extrahieren;
(b) Abtrennen des Heteropolywolframsäure enthaltenden Extraktionsmittels aus dem behandelten erschöpften Katalysator;
(c) Inkontaktbringen des Heteropolywolframsäure enthaltenden Extraktionsmittels mit einem stark sauren Ionenaustauscherharz zum Entfernen von Nickel, Cobalt, Chrom und Eisen, die aus dem darin enthaltenen erschöpften Katalysator in Schritt (a) ausgelaugt wurden und
(d) Rückgewinnen des Heteropolywolframsäure enthaltenden behandelten Extraktionsmittels zur späteren Verwendung.

2. Verfahren nach Anspruch 1, wobei die Heteropolysäure Silicowolframsäure oder Phosphorwolframsäure ist.

3. Verfahren nach Anspruch 1 oder Anspruch 2, **dadurch gekennzeichnet, dass** der erschöpfte Katalysator vor Schritt (a) mit einer organischen Flüssigkeit zum Entfernen von weichem Koks gewaschen wird.

4. Verfahren nach Anspruch 1, **dadurch gekennzeichnet, dass** Schritt (a) bei einer Temperatur im Bereich von 40 °C bis 85 °C durchgeführt wird.

5. Verfahren nach Anspruch 1, **dadurch gekennzeichnet, dass** das Extraktionsmittel ein Gemisch aus Ethanol und Wasser ist.

6. Verfahren nach Anspruch 1, **dadurch gekennzeichnet, dass** das Extraktionsmittel Wasser ist.

7. Verfahren nach Anspruch 1, **dadurch gekennzeichnet, dass** das Extraktionsmittel Ethanol ist, und Schritt (c) ein Schritt vorausgeht, in dem im Extraktionsmittel aufgelöster weicher Koks mittels einer Flüssig-Flüssig-Extraktion entfernt wird.

8. Verfahren nach Anspruch 1, **dadurch gekennzeichnet, dass** der erschöpfte Katalysator zur Bewirkung der Dehydratisierung von Ethoxyethan, Ethanol oder Gemischen davon zur Bildung von Ethen verwendet wurde.

9. Verfahren nach Anspruch 1, **dadurch gekennzeichnet, dass** rückgewonnene Heteropolywolframsäure zum Herstellen von frischer geträgerter Heteropolywolframsäure wieder verwendet wird.

10. Verfahren nach Anspruch 3, **dadurch gekennzeichnet, dass** die organische Flüssigkeit ein C₆- bis C₁₀-Alkan oder ein Gemisch davon ist.

## Revendications

1. Procédé de récupération d'acide hétéropolytungstique à partir d'un catalyseur usé comprenant un acide hétéropolytungstique supporté, **caractérisé en ce que** le procédé comprend :
(a) la mise en contact du catalyseur usé avec un extractant sélectionné parmi l'eau, le méthanol, l'éthanol ou un mélange de deux ou plusieurs de ceux-ci pendant suffisamment de temps pour extraire au moins une partie de l'acide hétéropolytungstique à partir dudit catalyseur ;
(b) la séparation de l'extractant contenant l'acide hétéropolytungstique du catalyseur usé traité ;
(c) la mise en contact de l'extractant contenant l'acide hétéropolytungstique avec une résine échangeuse d'ions acides forts pour enlever le nickel, le cobalt, le chrome et le fer éliminés par lessivage du catalyseur usé à l'étape (a) qui y sont présents, et
(d) la récupération de l'extractant traité contenant l'acide hétéropolytungstique pour une utilisation ultérieure.

2. Procédé selon la revendication 1, dans lequel l'hétéropolyacide est l'acide tungstosilicique ou l'acide tungstophosphorique.

3. Procédé selon la revendication 1 ou la revendication 2, **caractérisé en ce que** le catalyseur usé est lavé avant l'étape (a) avec un liquide organique pour enlever le coke mou.

4. Procédé selon la revendication 1, **caractérisé en ce que** l'étape (a) est exécutée à une température de 40 °C à 85 °C.

5. Procédé selon la revendication 1, **caractérisé en ce que** l'extractant est un mélange d'éthanol et d'eau.

6. Procédé selon la revendication 1, **caractérisé en ce que** l'extractant est de l'eau.

7. Procédé selon la revendication 1, **caractérisé en ce que** l'extractant est de l'éthanol, et **en ce que** l'étape (c) est précédée d'une étape dans laquelle le coke mou dissous dans l'extractant est éliminé en utilisant une extraction liquide-liquide.

8. Procédé selon la revendication 1, **caractérisé en ce que** le catalyseur usé a été utilisé pour effectuer la déshydratation d'éthoxyéthane, d'éthanol ou de mélanges de ceux-ci pour former de l'éthène.

9. Procédé selon la revendication 1, **caractérisé en ce que** l'acide hétéropolytungstique récupéré est réutilisé pour préparer de l'acide hétéropolytungstique supporté frais.

10. Procédé selon la revendication 3, **caractérisé en ce que** le liquide organique est un alcane C₆-C₁₀ ou un mélange de celui-ci.
